# EUROPEAN PATENT APPLICATION

(11) **EP 4 628 575 A1**
(43) Date of publication of application: **08.10.2025**
(21) Application number: 23903188.3
(22) Date of filing: 14.11.2023
(51) Int. Cl.: C12N 5/10, C12M 1/00, C12N 1/15, C12N 1/19, C12N 1/21, C12P 21/00

(54) **METHOD FOR CULTURING RECOMBINANT PROTEIN-PRODUCING CELL**

(30) Priority: 15.12.2022 JP 2022200442
(71) Applicant: Yamaha Hatsudoki Kabushiki Kaisha, Iwata-shi, Shizuoka 438-8501 (JP)
(72) Inventor: HIKICHI, Yuichi, Iwata-shi, Shizuoka 438-8501 (JP); HARADA, Gakuro, Iwata-shi, Shizuoka 438-8501 (JP); TSUJIOKA, Kazuya, Osaka-shi, Osaka 536-8523 (JP); TAHARA, Hiroshi, Osaka-shi, Osaka 536-8523 (JP)
(74) Representative: Müller-Boré & Partner Patentanwälte PartG mbB
(86) International application number: PCT/JP2023/040999
(87) International publication number: WO 2024/127890

(57) **Abstract**

A method for culturing a recombinant protein-producing cell includes the steps of: injecting a cell suspension, which contains a plurality of single cells capable of producing a recombinant protein in a culture medium, into a plate having a plurality of storage sections partitioned in minute sizes, and retaining the culture medium and one single cell in each of at least some of the plurality of storage sections; gelatinizing the culture medium in the storage sections; subjecting the single cell in each of the storage sections to a recombinant protein production period; and adding a liquid containing a detection protein capable of binding to a recombinant protein produced by the single cell in the gelatinized culture medium to the plate.

## Description

### Technical Field

The present invention relates to a method for culturing a cell producing a recombinant protein.

### Background Art

For the development of an antibody drug having an effect of specifically binding to an antigen of a foreign substance such as a virus-infected cell or a cancer cell and removing the foreign substance, it is essential to culture cells capable of producing an antibody. There are various prior art methods for culturing cells. For example, Patent Literature 1 discloses a culturing method using alginic acid gel as a spheroid culture technique.

Screening for identifying cells having a high antibody production amount from among the cultured cells is required. Patent Literature 2 discloses a screening method in which cells cultured in a plurality of wells are irradiated with light, and the antibody production amount is evaluated based on the amount of fluorescence generated from the wells. However, in a method in which cells are seeded in a general-purpose well and cultured to determine the amount of fluorescence, it may be difficult to efficiently perform screening.

### Citation List

### Patent Literature

Patent Literature 1: JP 2021-511078 A
Patent Literature 2: JP 6461580 B2

### Summary of Invention

An object of the present invention is to provide a method for culturing a recombinant protein-producing cell capable of efficiently identifying a cell strain producing a large amount of recombinant protein.

A method for culturing a recombinant protein-producing cell according to an aspect of the present invention includes the steps of: injecting a cell suspension, which contains a plurality of single cells capable of producing a recombinant protein in a culture medium, into a plate having a plurality of storage sections partitioned in minute sizes, and retaining the culture medium and one single cell in each of at least some of the plurality of storage sections; gelatinizing the culture medium in the storage sections; subjecting the single cell in each of the storage sections to a recombinant protein production period; and adding a liquid containing a detection protein capable of binding to a recombinant protein produced by the single cell in the gelatinized culture medium to the plate.

### Brief Description of Drawings

FIG. 1 is a view showing a step flow of a method for culturing antibody-producing cells according to an embodiment of the present invention.
FIG. 2A is a plan view with an enlarged view showing a structure of a first plate.
FIG. 2B is a cross-sectional view taken along line IIB-IIB of FIG. 2A.
FIG. 3 is a schematic cross-sectional view showing a situation in which cells are seeded on a first plate.
FIG. 4 is a schematic cross-sectional view showing a step of gelatinizing a culture medium in a microgrid.
FIG. 5 is a schematic cross-sectional view showing a step of primary culture of giving an antibody production period to a single cell.
FIG. 6A is a schematic cross-sectional view showing a situation in which a liquid culture medium containing a detection antibody is added to a first plate.
FIG. 6B is a schematic cross-sectional view showing a situation of cleaning.
FIG. 7A is a plan view showing a step of identifying a microgrid in which a single cell having a high antibody production amount is retained.
FIG. 7B is a plan view showing a step of identifying a microgrid in which a single cell having a high antibody production amount is retained.
FIG. 8A is a schematic cross-sectional view showing a situation of picking of a single cell by a suction tip.
FIG. 8B is a schematic view showing a step of transferring the picked single cells to the second plate.
FIG. 9 is a schematic cross-sectional view showing a step of ejecting a single cell from a suction tip to a second plate and retaining the single cell in a microgrid.
FIG. 10 is a schematic cross-sectional view 10 showing a step of establishing an extremely small culture environment by culture medium suction.
FIG. 11 is a schematic cross-sectional view showing a situation in which an opening of a microgrid is sealed with a sealing liquid.
FIG. 12 is an image showing the proliferation situation of single cells in secondary culture on a second plate.
FIG. 13 is an image of a proliferation situation of a single cell using the culturing method of a comparative example.
FIG. 14 is an image of a proliferation situation of a single cell using the culturing method of the comparative example.

### Description of Embodiments

Hereinafter, an embodiment of a method for culturing a recombinant protein-producing cell according to the present invention is described in detail with reference to the drawings. A target to be cultured in the present invention is a single cell producing a recombinant protein. In the following embodiment, as an example of the recombinant protein-producing cell, a single cell expected to be used in production of an antibody drug or antibody production, such as a CHO cell or a B cell, is exemplified. The culturing method shown in the present embodiment is generally a method in which the prepared single cells are cultured for a certain period, single cells excellent in antibody producing ability are selected, and the single cells are further cultured and proliferated to produce a large amount of antibodies.

### [Overall flow of culture step]

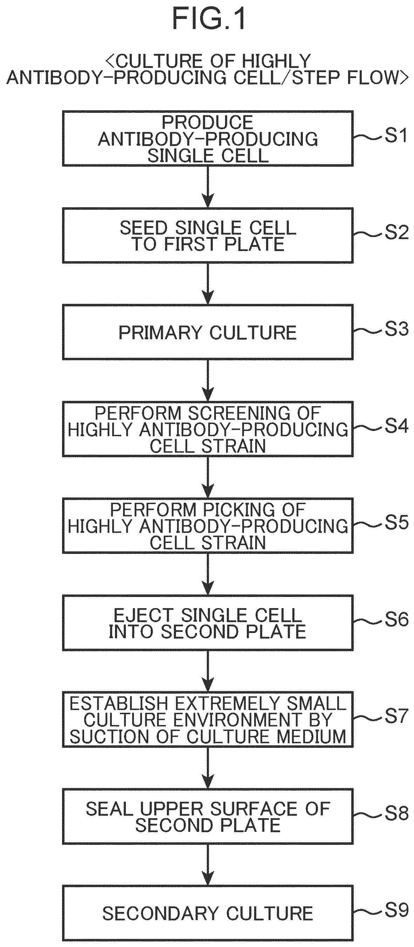

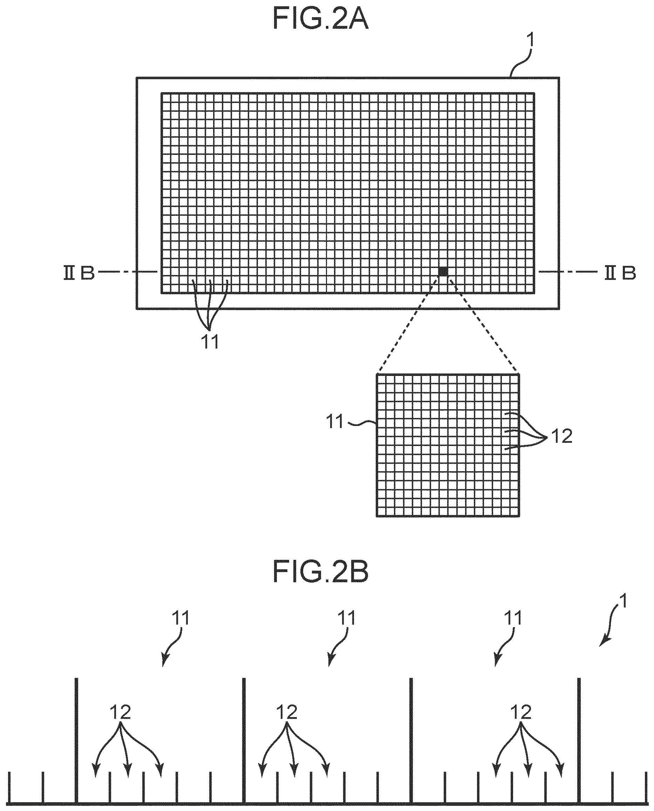

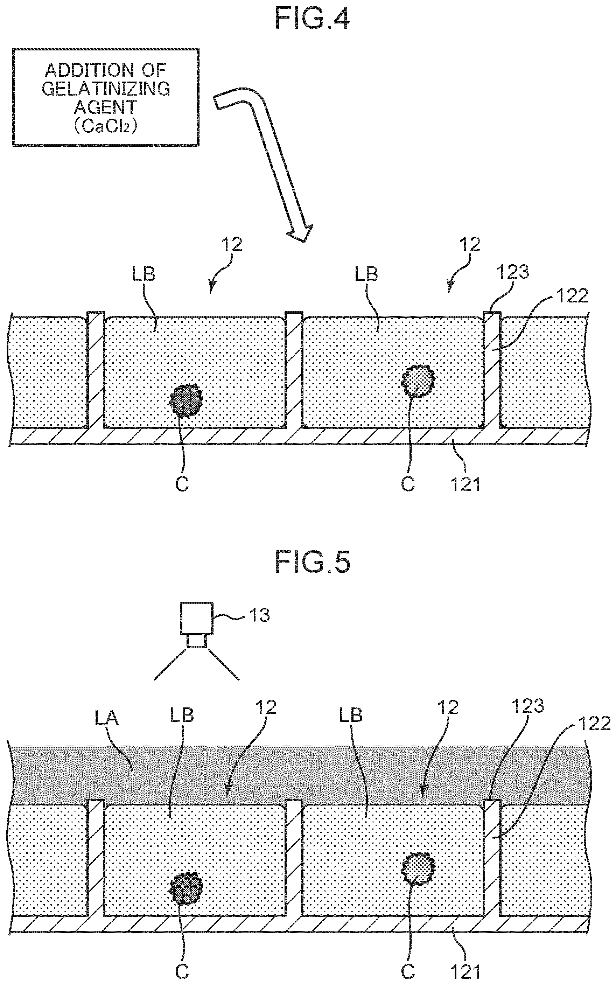

First, the overall flow of the method for culturing antibody-producing cells according to the present embodiment is described with reference to the step flow shown in FIG. 1. The culturing method of the present embodiment includes steps S1 to S9 sequentially performed. First, a large number of single cells capable of producing an antibody is produced using a predetermined method (step S1). Next, the produced single cells are seeded on the first plate 1 (FIG. 2A, FIG. 2B) having a plurality of cell storage sections together with the culture medium (step S2). Then, after gelatinizing the culture medium (FIG. 4), primary culture is performed in which single cells are cultured for a predetermined number of days in the cell storage section (step S3/FIG. 5).

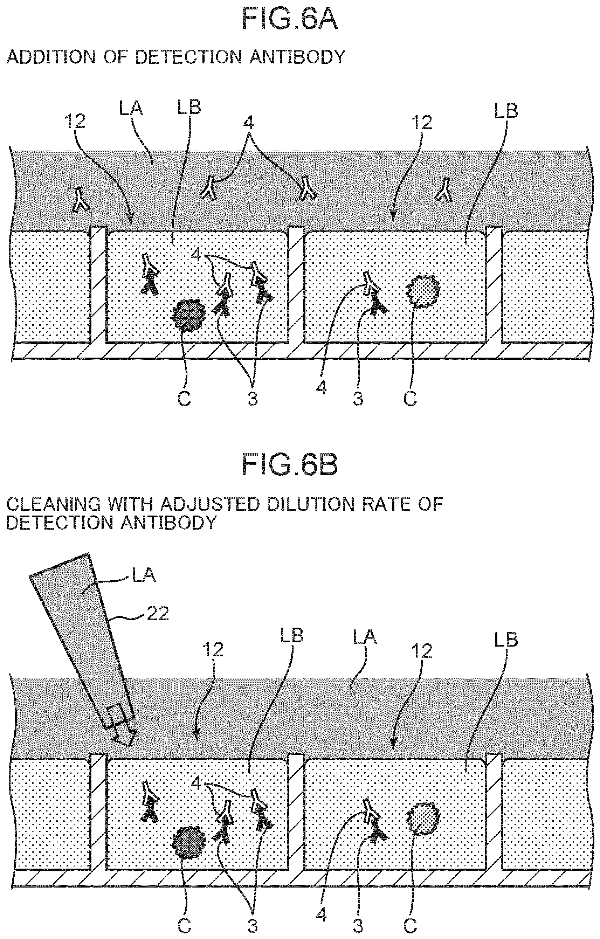

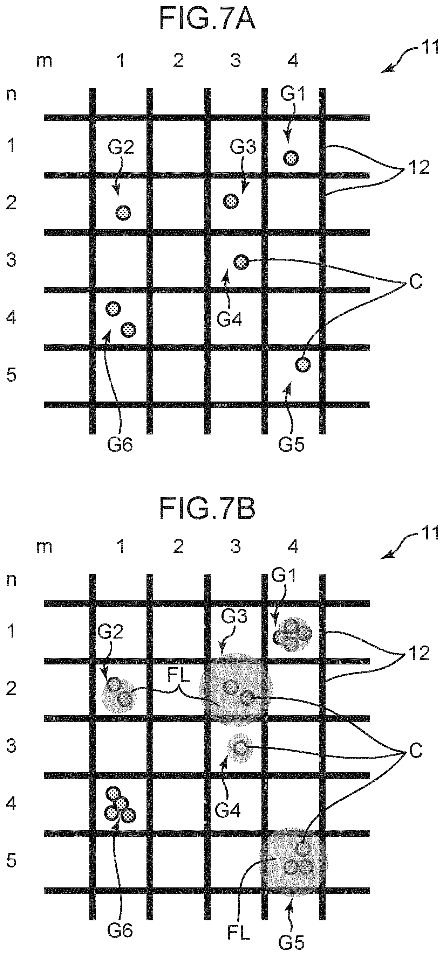

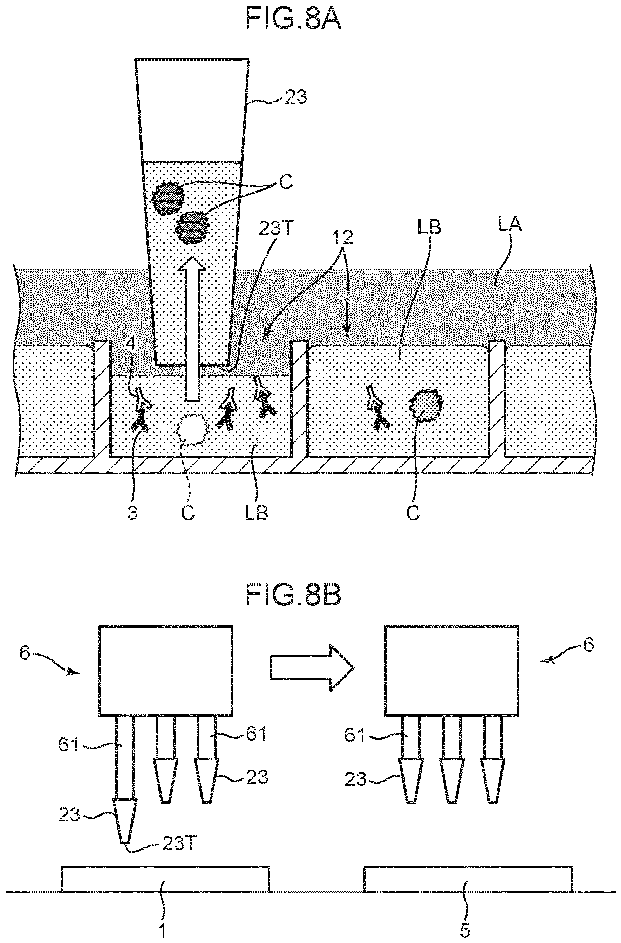

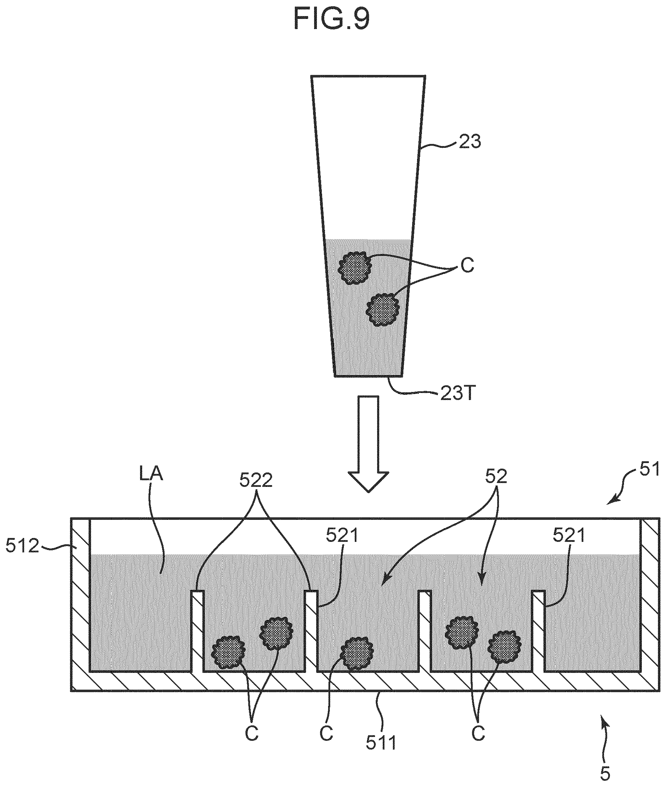

After the primary culture, a detection antibody that binds to the antibody produced by the single cell is added (FIG. 6A), and screening for identifying a highly antibody-producing cell strain (FIG. 7A, FIG. 7B) is performed (step S4). The identified highly antibody-producing cell strain is picked by a suction tip 23 and moved to a second plate 5 having a plurality of cell storage sections (step S5/FIG. 8A, FIG. 8B). Then, from the suction tip 23 to the second plate 5 on which the liquid culture medium is filled, single cells which are the picked highly antibody-producing cell strains are ejected (step S6/FIG. 9).

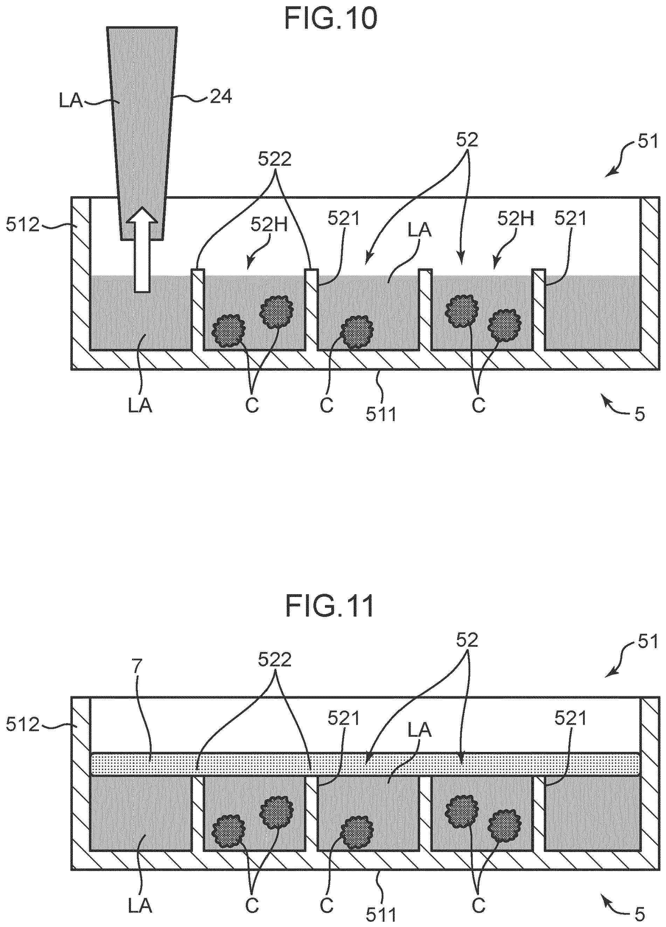

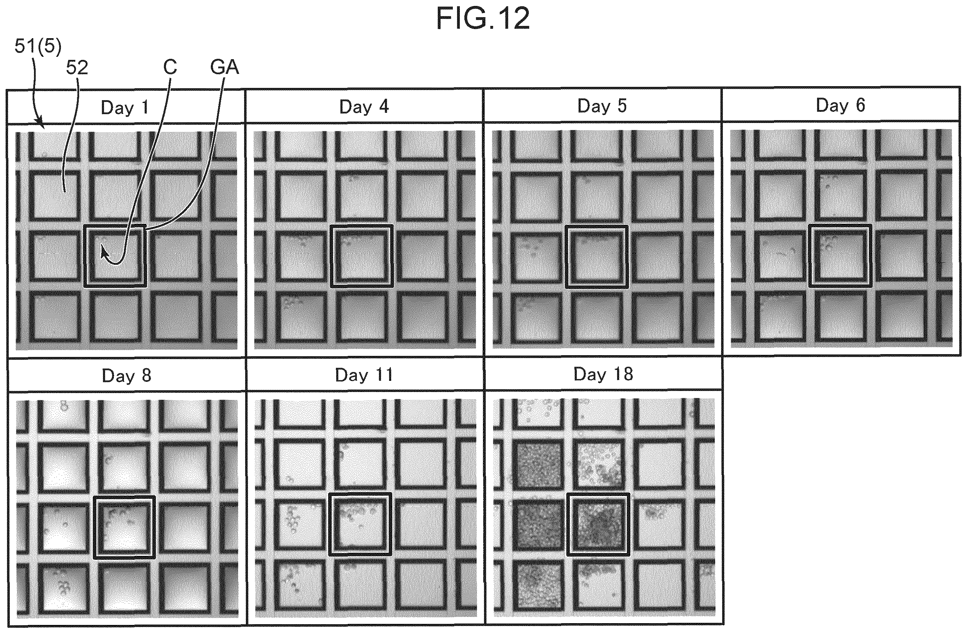

Subsequently, the liquid culture medium is suctioned from the second plate 5, and an extremely small culture environment in which single cells are cultured in individual cell storage sections, that is, a culture environment having an extremely small culture area is established (step S7/FIG. 10). Further, the upper surface of the second plate 5 is sealed with a sealing liquid for preventing evaporation of the culture medium (step S8/FIG. 11). Thereafter, secondary culture of culturing a single cell in the cell storage section for a predetermined number of days is performed (step S9/FIG. 12). Hereinafter, each of steps S1 to S9 described above is described in detail.

### [Step S1; Production of antibody-producing single cell]

In step S1, for example, by introducing a predetermined gene into a single cell to be cultured, the antibody producing ability is imparted to the single cell. A B cell of an immune cell can be exemplified as the single cell, and a monoclonal antibody produced by a single type of B cell can be exemplified as the antibody to be produced. The gene can be introduced by, for example, a chemical method such as transfection, a physical method such as electroporation, or a biological method such as a viral vector. It is desirable to employ a technique in which the single cell into which the gene has been introduced is cultured, for example, in a normal culture medium for two days, and then the culture medium is replaced with a selective culture medium suitable for the single cell to be cultured. Of course, the single cell may be cultured immediately in the selective culture medium after the gene introduction.

### [Step S2; Cell seeding on first plate]

In step S2, the single cells produced in step S1 are seeded on a culture plate for primary culture. FIG. 2A is a plan view with an enlarged view showing a structure of a first plate 1 (plate) as an example of the culture plate, and FIG. 2B is a cross-sectional view taken along line IIB-IIB of FIG. 2A. The first plate 1 includes grids 11 including recesses arranged in a matrix on one surface of a flat plate-shaped base material, and microgrids 12 (storage section) including recesses of minute sizes arranged in a matrix in each of the grids 11.

The grid 11 is a large partitioned part that divides a relatively large-sized region of the first plate 1. FIG. 2 illustrates a rectangular grid 11 partitioned by vertical and horizontal grid plates in top view. Instead of this, a structure in which the circular well-type grids 11 in top view are arranged in a honeycomb shape or a matrix shape may be adopted. The microgrid 12 is a small partitioned part that further subdivides the inside of each of the grids 11. The microgrid 12 is formed on a bottom plate of the grid 11, and is a recess that is rectangular in top view and is partitioned by a side plate lower than a grid plate partitioning the grid 11. The microgrid 12 may also be a circular well type in top view.

The microgrid 12 serves as a storage section that retains a single cell. As an example of the size of the microgrid 12, one side is 200 µm and the depth is 100 µm. The first plate 1 is a plate having a plurality of storage sections partitioned in such minute sizes. The microgrid 12 is desirably set to a size capable of forming a minute culture space, and can be set to, for example, a size selected from a range of an opening area of 4.0 × 10⁻² to 1.0 × 10⁻¹ mm² and a volume of 4.0 × 10⁻³ to 1.0 × 10⁻² mm³, more desirably a size selected from a range of an opening area of 1.0 × 10⁻³ to 1.0 × 10⁻¹ mm² and a volume of 2.0 × 10⁻⁵ to 1.0 × 10⁻² mm³.

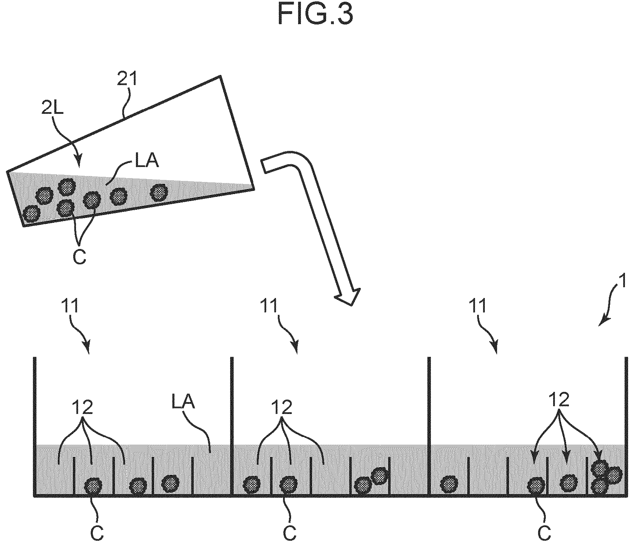

FIG. 3 is a schematic cross-sectional view showing a situation in which single cells C are seeded on the first plate 1. At the time of seeding, the cell suspension 2L is prepared in which a plurality of single cells C, produced in step S1, capable of producing an antibody are contained in a liquid culture medium LA. The cell suspension 2L is stored in the dispensing container 21 and injected into each grid 11 of the first plate 1. By this injection, the liquid culture medium LA and one single cell C are retained in at least some of a plurality of microgrids 12 in the grid 11. Of course, a microgrid 12 in which a plurality of single cells C enter or a single cell C is not retained also occurs. For example, in a case where there are 475 microgrids 12 in one grid 11, when 400 single cells are seeded in the grid 11, about 1/3 of the microgrids 12 stochastically become a grid retaining one single cell C.

In the cell suspension 2L, a gel material for immobilizing the liquid culture medium LA is blended in addition to the liquid culture medium LA. As the liquid culture medium LA, a normal culture solution containing a growth factor such as an inorganic salt, glucose, or amino acid and an additive component such as an antibiotic or a growth promoting factor in an aqueous medium may be used. For example, a CH150 culture medium (trade name of Gmep Incorporated) can be suitably used as the liquid culture medium LA. The gel material is not particularly limited as long as the liquid culture medium LA can be immobilized, and it is desirable to use alginate. The liquid culture medium LA containing alginate has a property of being quickly gelatinized by adding, for example, a gelatinizing agent containing a calcium ion or a magnesium ion, and thus has an advantage that the subsequent gelatinizing step can be quickly completed.

### [Step S3; Primary culture]

Step S3 is a step of gelatinizing the liquid culture medium LA in the microgrid 12, then culturing the single cell C in the microgrid 12 for a certain period of time, and giving the single cell an antibody production period. FIG. 4 is an enlarged cross-sectional view of the microgrid 12, and is a schematic view showing a step of gelatinizing the liquid culture medium LA in the microgrid 12. Each microgrid 12 is partitioned by a bottom plate 121 and a side plate 122.

The liquid culture medium LA containing alginate is injected into the first plate 1, and then a gelatinizing agent is added thereto. As the gelatinizing agent, for example, CaCl₂ can be used. By the addition of the gelatinizing agent, the liquid culture medium LA in the microgrid 12 becomes a gel culture medium LB. The single cell C in microgrid 12 is immobilized by gel culture medium LB. The height of the gel culture medium LB is desirably adjusted to be the same as or slightly lower than the height of the top part 123 of the side plate 122. As a result, a state in which one single cell C is confined in the gel culture medium LB of one microgrid 12 can be formed. The height of the gel culture medium LB may be slightly higher than the top part 123, or the gel culture media LB of the adjacent microgrids 12 may be connected to each other.

FIG. 5 is a schematic cross-sectional view showing a step of primary culture of giving an antibody production period to a single cell. A gelatinizing agent is added, and the mixture is allowed to stand for a certain period of time (for example, 30 minutes) to convert the liquid culture medium LA into the gel culture medium LB, and then the liquid culture medium LA is injected into the first plate 1. That is, the upper side of the microgrid 12 provided with the gel culture medium LB is covered with the liquid culture medium LA. In this state, by primarily culturing the single cell C only for a predetermined antibody production period, it is possible to cause the single cell C to perform cell proliferation and antibody production for each microgrid 12. A camera 13 is disposed on the upper surface of the first plate 1. During the primary culture period, the microgrid 12 of the first plate 1 is imaged by the camera 13 and state monitoring is performed. The period of the primary culture is, for example, about 4 to 8 days.

FIG. 6A is a diagram schematically showing a state in which several days have elapsed from the primary culture. Here, a state in which the single cell C in the microgrid 12 has produced an antibody 3 is shown. During the primary culture, the liquid culture medium LA is replaced. The replacement liquid culture medium LA contains a detection antibody 4 capable of binding to the antibody 3 produced by the single cell C in the gel culture medium LB. FIG. 6A shows a state in which a liquid culture medium LA containing the detection antibody 4 is added to the first plate 1, and a part of the detection antibody 4 is bound to the antibody 3.

The replacement of the liquid culture medium LA is performed in a mode of cleaning in which the liquid culture medium LA already injected into the first plate 1 is absorbed, and the liquid culture medium LA containing the detection antibody 4 is newly injected. FIG. 6B is a schematic cross-sectional view showing a situation of the cleaning. First, the initial liquid culture medium LA illustrated in FIG. 5 is suctioned by a suction tip (not illustrated). Thereafter, a replacement liquid culture medium LA containing the detection antibody 4 is prepared. The replacement liquid culture medium LA is retained by the culture medium supply tip 22 and injected onto the gel culture medium LB of the microgrid 12 as illustrated in FIG. 6B. Such cleaning is performed about 1 to 3 times during the primary culture.

The dilution rate of the detection antibody 4 in the replacement liquid culture medium LA is desirably selected within a range in which an excess amount that cannot be bound to the antibody 3 does not excessively occur. When the detection antibody 4 is excessively contained, many detection antibodies 4 float in the liquid culture medium LA on the upper layer of the gel culture medium LB, and the production amount of the antibody 3 of each single cell C may not be accurately evaluated.

### [Step S4; Screening of highly antibody-producing cell strain]

Step S4 is a step of giving a trigger for reacting the detection antibody 4 added in step S3 and identifying the microgrid 12 retaining the single cell C having a high antibody production amount. In the present embodiment, an example is shown in which the first plate 1 is irradiated with light as the trigger to cause the detection antibody 4 to fluoresce. In the microgrid 12 retaining the single cell C in which the production amount of the antibody 3 is large, the detection antibody 4 binding to the antibody 3 also increases, and the degree of fluorescence increases. Therefore, a cell strain having a high antibody production amount can be identified only by evaluating the degree of fluorescence of the detection antibody 4 in units of the microgrids 12.

FIG. 7A and FIG. 7B are plan views showing a step of identifying the microgrid 12 in which the single cell C having a high antibody production amount is retained. FIG. 7A shows the retaining situation of the single cell C in the microgrid 12 at an early stage of primary culture, which is the antibody production period. Addresses of n rows and m columns are assigned to the microgrids 12 arranged in a matrix. Among the microgrids 12 of n rows and m columns, one single cell C is retained in each of a grid G1 of n1m4, a grid G2 of n2m1, a grid G3 of n2m3, a grid G4 of n3m3, and a grid G5 of n5m4. In the grid G6 of n4m1, a plurality of (two) single cells C are retained from the beginning of the primary culture.

FIG. 7B shows a state after completion of the primary culture. Except for the grid G4, the single cell C is proliferating. FIG. 7B shows a state in which the first plate 1 is irradiated with light of a predetermined wavelength from a light source (not illustrated), and fluorescence FL is generated from the detection antibody 4. In the grid G3 and the grid G5, the fluorescence FL having a light emission area exceeding the opening area of the microgrid 12 is generated, and it is found that the single cell C having a high antibody production amount is cultured. On the other hand, for the grid G1, although the single cell C has proliferated to 4 cells, only fluorescence FL having a small emission area with respect to the opening area of the microgrid 12 is generated. The fluorescence FL of the grids G2 and G4 is also small.

The antibody production amount is evaluated by the degree of generation of fluorescence FL for each microgrid 12. As the evaluation index, for example, (1) the generation range of the fluorescence FL, (2) the average luminance of the fluorescence FL, and (3) the maximum luminance of the fluorescence FL can be exemplified. The generation range of the fluorescence FL is a ratio of the emission area of the fluorescence FL generated from the microgrid 12 to the opening area of the microgrid 12. In the example of FIG. 7B, the fluorescence FL of the grids G3 and G5 has a light emission area exceeding the opening area of each microgrid 12, and is evaluated as a high antibody production amount. On the other hand, the emission areas of the fluorescence FL of the grids G1, G2, and G4 are narrow, and the antibody production amounts thereof are low. Based on the generation range of the fluorescence FL, the average luminance and the maximum luminance are further considered. The antibody production amount of a grid having higher average luminance and maximum luminance is highly evaluated. Here, grids G3 and G5 may be identified as "microgrids 12 in which highly antibody-producing cell strains are retained".

Note that the grid G6 in which the plurality of single cells C are retained from the beginning of the antibody production period is excluded from the identification target. In the over-retained grid in which a plurality of single cells C are retained in one microgrid 12, there is a possibility that a highly antibody-producing cell strain and a non-producing or low antibody-producing cell strain are mixed. In this case, the latter may be picked in the subsequent step S5. Therefore, the first plate 1 is monitored by the camera 13 (FIG. 5) from the beginning of the primary culture, and an over-retained grid such as the grid G6 is excluded from the evaluation target of the antibody production amount even if fluorescence FL having a high degree of occurrence is generated. This makes it possible to accurately identify the microgrid 12 retaining the single cell C having a high antibody production amount.

### [Step S5; Picking of highly antibody-producing cell strain]

In step S5, the single cell C stored in the microgrid 12 identified as the grid having a high antibody production amount in the previous step S4 is picked by the suction tip 23 and moved to the second plate 5. That is, picking is performed in units of the microgrids 12. FIG. 8A is a schematic cross-sectional view showing a situation of picking of the single cell C by the suction tip 23, and FIG. 8B is a schematic view showing a step of transferring the picked single cell C to the second plate 5.

The suction tip 23 includes a tip opening 23T for suctioning and ejecting the single cell C at the lower end. As illustrated in FIG. 8A, the tip opening 23T of the suction tip 23 is aligned in the XY direction with respect to the single cell C of the suction target, and the suction tip 23 is lowered such that the tip opening 23T enters the microgrid 12. Thereafter, by generating a negative pressure in the tip opening 23T, the single cell C of the suction target is suctioned into the suction tip 23 together with the gel culture medium LB. The XY coordinates of the single cell C can be obtained based on a photographed image of the first plate 1 by the camera 13 (FIG. 5). In the suction by the suction tip 23, all the single cells C presenting in one microgrid 12 identified as the high antibody production amount may be suctioned by a suction operation of one turn, or a part thereof may be suctioned.

As illustrated in FIG. 8B, the suction tip 23 is attached to a head 61 of a head unit 6. The head unit 6 is a unit movable in a horizontal direction (XY direction) along a guide rail (not illustrated). The head 61 is attached to the main body of the head unit 6 so as to be movable up and down, and has a lower end part to which the suction tip 23 is attached. In the main body of the head unit 6, a mechanism for generating negative pressure and positive pressure at the lower end part of the head 61 is built.

By the XY movement of the head unit 6, the tip opening 23T of the suction tip 23 and the single cell C of the suction target are aligned. Advancing and retracting of the tip opening 23T with respect to the microgrid 12 retaining the single cell C of the suction target is achieved by raising and lowering of the head 61. Suction or ejection of the single cell C from the tip opening 23T is achieved by generating a negative pressure or a positive pressure at the lower end part of the head 61. In the first plate 1, when the single cell C of the suction target is suctioned by the suction tip 23 attached to each head 61, the head unit 6 is moved to above the second plate 5 (another container) on which the secondary culture is performed. As a device that automatically performs the cell picking and the cell movement as described above, for example, CELL HANDLER (trade name of YAMATO MOTOR CO., LTD.) can be suitably used. Instead of such an automated device, an operator may manually perform cell picking and cell movement using a cell suction/ejection tool such as a micropipette.

### [Step S6; Cell ejection to second plate]

Step S6 is a step of retaining the single cell C picked by the suction tip 23 in step S5 in a predetermined storage section of the second plate 5. FIG. 9 is a schematic cross-sectional view showing a step of retaining the single cell C in the second plate 5 by the ejection in step S6. The step S6 includes a step of injecting a predetermined amount of liquid culture medium into the second plate 5 in advance, and a step of ejecting the single cell C picked by the first plate 1 to the second plate 5.

The second plate 5 is a container including a plurality of storage sections having openings on the upper surface. As the second plate 5, a plate having the same structure as the first plate 1 described above with reference to FIG. 2 can be used. FIG. 9 illustrates the second plate 5 including a grid 51 that divides a relatively large-sized region of the second plate 5 and a microgrid 52 that further subdivides the inside of the grid 51. Each microgrid 52 is the storage section that retains the single cell C. FIG. 9 shows a cross-sectional view of one grid 51. The grid 51 includes a grid bottom plate 511 forming a bottom surface and a grid side plate 512 forming a side surface. Each microgrid 52 is partitioned by a common grid bottom plate 511 and side plates 521 forming individual side surfaces.

It is desirable that the microgrid 52 serving as a storage section for retaining the single cell C has the same size as the microgrid 12 of the first plate 1. That is, the microgrid 52 is desirably set to a size capable of forming a minute culture space, and can be set to, for example, a size selected from a range of an opening area of 4.0 × 10⁻² to 1.0 × 10⁻¹ mm² and a volume of 4.0 × 10⁻³ to 1.0 × 10⁻² mm³, more desirably a size selected from a range of an opening area of 1.0 × 10⁻³ to 1.0 × 10⁻¹ mm² and a volume of 2.0 × 10⁻⁵ to 1.0 × 10⁻² mm³.

Prior to ejection of the single cells C, a predetermined amount of the liquid culture medium LA is input into the second plate 5. As illustrated in FIG. 9, the input amount of the liquid culture medium LA is an amount by which the liquid level reaches above the top part 522 of the side plate 521 partitioning the microgrid 52. In other words, the amount of the liquid culture medium LA whose liquid level is located above the upper surface opening of the microgrid 52 is injected into the grid 51 of the second plate 5 in advance. Note that the top parts 522 of the microgrids 52 are at the same height position.

Thereafter, from the suction tip 23, the single cells C for one microgrid 12 sorted as the highly antibody-producing cell strain are ejected to the second plate 5. As illustrated in FIG. 9, this ejection is achieved by causing the tip opening 23T of the suction tip 23 to face the opening of the grid 51 and generating a positive ejection pressure in the tip opening 23T. Of course, the ejection may be performed by rushing the tip opening 23T into the upper layer part of the liquid culture medium LA in the grid 51, or the ejection may be performed by entering the tip opening 23T into the microgrid 52. By this ejection, among the plurality of the microgrids 52 included in one grid 51, one or more single cells C are retained in at least some of the microgrids 52.

### [Step S7; Establishment of extremely small culture environment by culture medium suction]

Step S7 is a step of suctioning the liquid culture medium LA of the second plate 5 to establish a culture environment of an independent single cell C in units of the microgrids 52. The culture environment established here is a culture environment having an extremely small culture area. FIG. 10 is a schematic cross-sectional view showing the operation of step S7. FIG. 10 shows a state in which the liquid culture medium LA in the grid 51 is suctioned by a liquid absorbing tip 24.

The suction by the liquid absorbing tip 24 is performed from the state of FIG. 9 until the liquid level of the liquid culture medium LA of the grid 51 is in a state where the top part 522 of the side plate 521 of the microgrid 52 is exposed. That is, the liquid culture medium LA of the grid 52 is removed until the liquid level of the liquid culture medium LA substantially coincides with the height position of an opening 52H of the microgrid 51. By such suction, the liquid culture medium LA in one microgrid 52 and the liquid culture medium LA in the other microgrid 52 are not mixed. That is, a culture environment consisting of the liquid culture medium LA in the individual microgrids 52 and having an extremely small culture area for the single cell C is formed. The amount of the liquid culture medium LA in one microgrid 52 is, for example, 4 nanoliter.

In an extremely small culture environment in which a small number of single cells C of about 1 to 10 are input into a vast culture environment, the single cells C are difficult to proliferate. For example, in FIG. 10, even if the liquid culture medium LA is injected into the grid 51 in which the side plate 521 is removed and the partition of the microgrid 52 is eliminated, and the single cell C is input and given a predetermined culture period, the single cell C hardly proliferates. On the other hand, when about 1 to 10 single cells C are input and cultured in a culture environment in which the liquid culture medium LA is about 4 nanoliters, the cells are likely to be adjacent to each other, and the proliferation of the single cells C tends to be promoted. In the cell ejection in step S6, it is advantageous that the liquid level of the liquid culture medium LA is higher than the opening 52H because the single cell C can be retained in the microgrids 52 in one ejection operation. By suctioning the culture medium in the subsequent step S7, it is possible to establish a culture environment suitable for culture and proliferation of a small number of single cells C isolated in units of the microgrids 52.

### [Step S8; Sealing of microgrid]

Step S8 is a step of injecting a sealing liquid 7 into the second plate 5 to seal the upper side of the opening 52H of the microgrid 52. FIG. 11 is a schematic cross-sectional view showing a situation where the opening 52H of the microgrid 52 is sealed with the sealing liquid 7. The lower surface of the sealing liquid 7 is in contact with the top part 522 of the side plate 521 of the microgrid 52 and closes the opening part 52H. That is, the liquid culture medium LA and the single cell C are confined in one microgrid 52 by the sealing liquid 7. As the sealing liquid 7, for example, an embryo culture oil made of light liquid paraffin or the like can be used.

The function required as the sealing liquid 7 is an evaporation preventing function of the liquid culture medium LA in the microgrid 52. Since the liquid culture medium LA contains moisture, the moisture evaporates when the sealing liquid 7 is not present. For this reason, during the secondary culture in step S9, the amount of the liquid culture medium LA in the microgrid 52 decreases or is depleted, or a failure such as a change in the culture medium state such as osmotic pressure or pH occurs. By sealing the opening 52H with the sealing liquid 7 having an evaporation preventing function, evaporation of the liquid culture medium LA during the secondary culture period can be suppressed.

Another desirable function of the sealing liquid 7 is air permeability. With the sealing liquid 7 having air permeability, even if the opening 52H of the microgrid 52 is sealed, the liquid culture medium LA in the microgrid 52 can be communicated with the atmosphere. Therefore, the culture environment of the single cell C in the microgrid 52 can be maintained healthy. The embryo culture oil described above has both the evaporation preventing function and the air permeability, and thus is suitable as the sealing liquid 7. Other than the embryo culture oil, other liquid or semi-liquid (gel) having at least an evaporation preventing function may be used as the sealing liquid 7. Needless to say, it is necessary that the specific gravity is smaller than that of the liquid culture medium LA.

By forming the layer of the sealing liquid 7, the extremely small culture environment established in step S7 can be maintained during the secondary culture period. That is, not only evaporation of the liquid culture medium LA in the microgrid 52 can be prevented, but also contamination of foreign substance contained in the outside air, for example, minute dust, mold spores, bacteria, and the like into the microgrid 52 can be suppressed. There is also an advantage that diffusion of the active substance emitted by the single cell C cultured in the microgrid 52 can be prevented and the proliferation of the single cell C can be promoted.

### [Step S9; Secondary culture]

Step S9 is a step of culturing the single cell C for a predetermined culture period in a state where the opening 52H of the microgrid 52 is sealed with the sealing liquid 7 as illustrated in FIG. 11. That is, the step is a step of producing a large amount of the antibodies 3 by further secondarily culturing and proliferating the single cell C identified as the highly antibody-producing cell strain in step S4 for a predetermined period. During the secondary culture, a liquid culture medium containing growth factors is supplemented in the grid 51.

FIG. 12 is an image showing the proliferation situation of the single cell C in the secondary culture on the second plate 5. "Day 1" in the figure means a state on the first day from the start of the secondary culture. FIG. 12 shows images of a part of the microgrid 52 included in one grid 51 on Day 1, Day 4, Day 5, Day 6, Day 8, Day 11, and Day 18 from the start of the secondary culture. When the mutation situation of the single cell C in a target grid GA among the plurality of the microgrids 52 is observed, it is found that the single cell C is proliferated every day. Incidentally, the reason why the single cells C around the target grid GA also proliferate rapidly between Day 11 and Day 18 is that the number of culture days is simply long, and in addition, the single cells C proliferated from the target grid GA entered the adjacent grid when the liquid culture medium is supplemented.

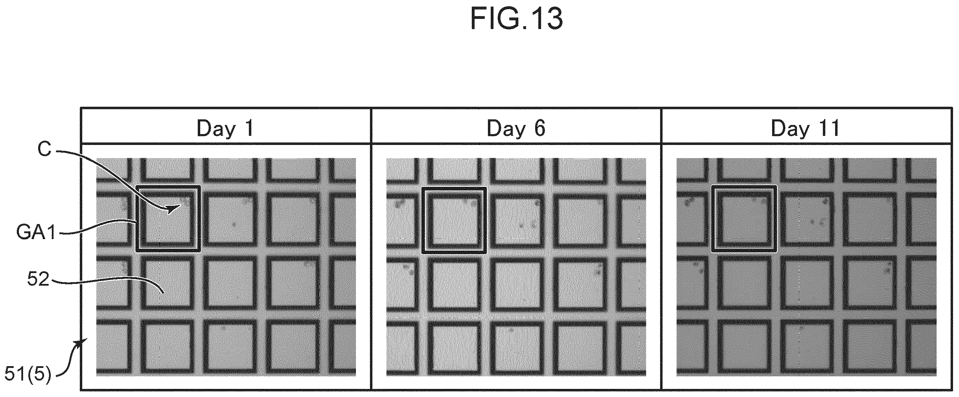

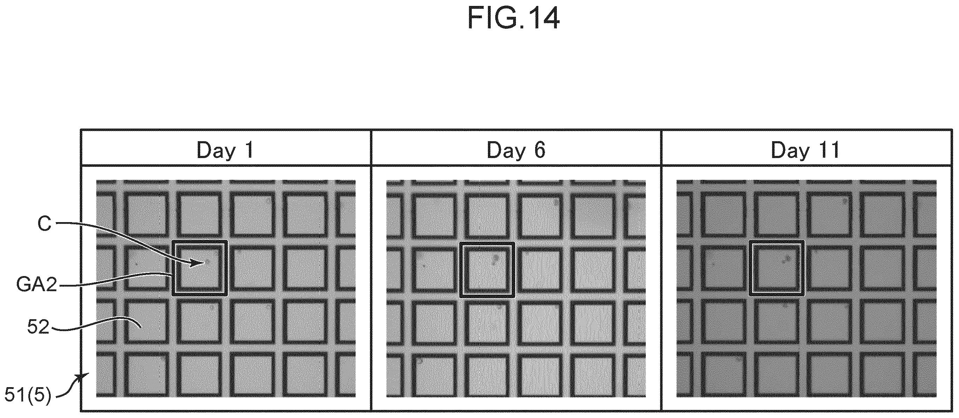

FIG. 13 and FIG. 14 are images of the proliferation situation of single cells using the culturing method of Comparative Example. FIG. 13 is an image of Comparative Example 1 in which the secondary culture is immediately performed in a state in which the culture medium suction in step S7 (FIG. 10) is not performed and the sealing with the embryo culture oil in step S8 (FIG. 11) is not performed, that is, after the cell ejection in step S6 (FIG. 9). In FIG. 13, images on Day 1, Day 6, and Day 11 from the start of the secondary culture are shown. Looking at the proliferation situation of the single cell C in the target grid GA1 among the plurality of the microgrids 52, it can be seen that no significant proliferation occurred between Day 1 and Day 11.

FIG. 14 is an image of Comparative Example 2 in which the secondary culture was performed by injecting the sealing liquid 7 in a state in which the sealing with the embryo culture oil in step S8 was performed without performing the culture medium suction in step S7 (FIG. 10), that is, in a state in which the liquid level of the liquid culture medium LA is at a position higher than the top part 522 after the cell ejection in step S6 (FIG. 9). FIG. 14 also shows images on Day 1, Day 6, and Day 11 from the start of the secondary culture. Looking at the proliferation situation of the single cell C in the target grid GA2 among the plurality of the microgrids 52, it can be seen that no significant proliferation occurred between Day 1 and Day 11.

### [Operation and effect]

The method for culturing an antibody-producing cell according to the present embodiment described above has the following functional effect. First, in the primary culture before picking in step S5, after one single cell C is retained in one microgrid 12 of the first plate 1, the liquid culture medium LA in the microgrid 12 is gelatinized. By gelation of the liquid culture medium LA, a state in which one single cell C is confined in one microgrid 12 can be formed. In this state, by primarily culturing the single cell C for a predetermined antibody production period, cell proliferation and production of the antibody 3 can be performed for each microgrid 12. Then, the antibody production amount can be evaluated in units of the microgrids 12 by adding the detection antibody 4 to the first plate 1. Since floating of the single cell C is suppressed by gelation of the culture medium, a highly antibody-producing cell strain is retained in the microgrid 12 in which a large amount of the detection antibodies 4 are detected. As described above, since it is possible to culture the single cell C, produce the antibody 3, and detect the antibody production amount in units of the microgrids 12, a cell strain having a high antibody production amount can be efficiently identified.

Next, in the secondary culture after picking in step S5, after the single cell C is retained by the microgrid 52 of the second plate 5, the liquid culture medium LA is removed, and the opening 52H is sealed with the sealing liquid 7. This makes it possible to construct an extremely small culture environment closed in units of the microgrids 52 while preventing evaporation of the liquid culture medium LA in the microgrids 52. Culturing a single cell C in a small culture region promotes the proliferation of the single cell C. Therefore, by picking the single cell C having excellent antibody producing ability and secondarily culturing the single cell C as described above, the efficiency of proliferation of the single cell C can be improved, and furthermore, a large amount of the antibodies 3 can be produced.

### [Inventions included in the above embodiments]

The embodiment described above includes the invention having the following configuration.

A method for culturing a recombinant protein-producing cell according to an aspect of the present invention includes the steps of: injecting a cell suspension, which contains a plurality of single cells capable of producing a recombinant protein in a culture medium, into a plate having a plurality of storage sections partitioned in minute sizes, and retaining the culture medium and one single cell in each of at least some of the plurality of storage sections; gelatinizing the culture medium in the storage sections; subjecting the single cell in each of the storage sections to a recombinant protein production period; and adding a liquid containing a detection protein capable of binding to a recombinant protein produced by the single cell in the gelatinized culture medium to the plate.

According to this aspect, after one single cell is retained in one storage section, the culture medium in the storage section is gelatinized. By gelation, a state in which one single cell is confined in one storage section can be formed. In this state, by culturing the single cell for a predetermined recombinant protein production period, cell proliferation and recombinant protein production can be performed for each storage section. Then, by adding the detection protein to the plate, the production amount of the recombinant protein can be evaluated in units of storage sections. Since floating of a single cell is suppressed by gelation of the culture medium, a cell strain having high recombinant protein production ability is retained in the storage section in which a large amount of detection protein is detected. As described above, according to the above aspect, it is possible to culture single cells, produce recombinant protein, and detect the amount of recombinant protein produced in units of storage sections, and thus it is possible to efficiently identify a cell strain having a large amount of recombinant protein produced.

In the above culturing method, it is desirable that the single cell capable of producing the recombinant protein is a single cell capable of producing an antibody, and the detection protein is a detection antibody.

According to this aspect, since it is possible to culture a single cell, produce an antibody, and detect the antibody production amount in units of storage sections, a cell strain having a high antibody production amount can be efficiently identified.

The above culturing method desirably further includes a step of providing a trigger for reacting the detection antibody, and identifying a storage section in which a single cell having a high antibody production amount is retained.

According to this aspect, it is possible to identify a cell strain having a high antibody production amount only by applying a trigger such as irradiating the plate with light to fluoresce the detection antibody and detecting the amount of the detection antibody in units of storage sections based on the degree of fluorescence or the like.

The above culturing method may further include a step of picking the single cell retained in the identified storage section and transferring the single cell to another container.

According to this aspect, by picking and transferring the highly antibody-producing cell strain to another container, for example, the cell strain can be further cultured to produce a large amount of antibodies.

In the above culturing method, it is desirable that the plate is monitored during the production period of the antibody, and the storage section in which a plurality of single cells are retained from beginning of the production period is excluded from a target of the identification.

When the cell suspension is injected into the plate, a storage section retaining only one single cell at a stochastically substantially constant rate among a plurality of storage sections is formed. The remaining storage section is a non-retaining storage section that does not retain a single cell or an over retaining storage section that retains a plurality of single cells. In the over retaining storage section, there is a possibility that a highly antibody-producing cell strain and a non-producing or low antibody-producing cell strain are mixed. According to the above aspect, since the over retaining storage section is excluded from the identification target, it is possible to accurately identify the storage section retaining the single cell having a high antibody production amount.

In the above culturing method, it is desirable that the storage section has a size selected from a range of an opening area of 1.0 × 10⁻³ to 1.0 × 10⁻¹ mm² and a volume of 2.0 × 10⁻⁵ to 1.0 × 10⁻² mm³.

According to this aspect, a culture region of single cells can be sufficiently miniaturized, and a large number of single cells can be independently cultured with a limited plate area. Therefore, it is possible to improve the efficiency of culture and the efficiency of antibody production amount evaluation.

In the above culturing method, it is preferable that the plate includes a large partitioned part that divides a relatively large-sized region of the plate, and a small partitioned part that further subdivides an inside of the large partitioned part, and the small partitioned part is the storage section.

According to this aspect, it is possible to utilize it by, for example, changing a cell type or a culture solution in units of large partitioned parts, and culture can be diversified.

In the above culturing method, it is desirable that a culture medium containing alginate is injected into the plate.

The alginate-containing culture medium has a property of quickly gelatinizing, for example, by adding a calcium ion or a magnesium ion. Therefore, according to the above aspect, the gelation step can be completed quickly.

The above culturing method desirably further includes a step of introducing a gene into the single cell to impart antibody producing ability, and after the single cell into which the gene has been introduced is cultured in a normal culture medium for a predetermined period, the normal culture medium is replaced with a selective culture medium suitable for culturing the single cell.

According to this aspect, although the cause has not been ascertained, a single cell having antibody producing ability can be satisfactorily proliferated.

According to the present invention described above, it is possible to provide a culturing method capable of efficiently identifying a cell strain in which the production amount of a recombinant protein is large.

## Claims

1. method for culturing a recombinant protein-producing cell, the method comprising the steps of:
injecting a cell suspension, which contains a plurality of single cells capable of producing a recombinant protein in a culture medium, into a plate having a plurality of storage sections partitioned in minute sizes, and retaining the culture medium and one single cell in each of at least some of the plurality of storage sections;
gelatinizing the culture medium in the storage sections;
subjecting the single cell in each of the storage sections to a recombinant protein production period; and
adding a liquid containing a detection protein capable of binding to a recombinant protein produced by the single cell in the gelatinized culture medium to the plate.

2. The method for culturing a recombinant protein-producing cell according to claim 1, wherein
the single cell capable of producing the recombinant protein is a single cell capable of producing an antibody, and
the detection protein is a detection antibody.

3. The method for culturing a recombinant protein-producing cell according to claim 2, wherein the method further comprises a step of providing a trigger for reacting the detection antibody, and identifying a storage section in which a single cell having a high antibody production amount is retained.

4. The method for culturing a recombinant protein-producing cell according to claim 3, wherein the method further comprises a step of picking the single cell retained in the identified storage section and transferring the single cell to another container.

5. The method for culturing a recombinant protein-producing cell according to claim 3, wherein the plate is monitored during the production period of the antibody, and the storage section in which a plurality of single cells are retained from beginning of the production period is excluded from a target of the identification.

6. The method for culturing a recombinant protein-producing cell according to any one of claims 1 to 5, wherein the storage section has a size selected from a range of an opening area of 1.0 × 10⁻³ to 1.0 × 10⁻¹ mm² and a volume of 2.0 × 10⁻⁵ to 1.0 × 10⁻² mm³.

7. The method for culturing a recombinant protein-producing cell according to claim 6, wherein
the plate includes a large partitioned part that divides a relatively large-sized region of the plate, and a small partitioned part that further subdivides an inside of the large partitioned part, and
the small partitioned part is the storage section.

8. The method for culturing a recombinant protein-producing cell according to any one of claims 2 to 5, wherein a culture medium containing alginate is injected into the plate.

9. The method for culturing a recombinant protein-producing cell according to any one of claims 2 to 5, wherein
the method further comprises a step of introducing a gene into the single cell to impart antibody producing ability, and
after the single cell into which the gene has been introduced is cultured in a normal culture medium for a predetermined period, the normal culture medium is replaced with a selective culture medium suitable for culturing the single cell.
